# EUROPEAN PATENT SPECIFICATION

(11) **EP 0 444 546 B1**
(45) Date of publication and mention of the grant of the patent: **11.09.1996**
(21) Application number: 91102632.6
(22) Date of filing: 22.02.1991
(51) Int. Cl.: A61K 31/415

(54) **Hydantoin derivatives for use as hypoglycemic and/or hypolipidemic agents**
Derivate des Hydantoins zur Verwendung als hypoglycemische und/oder hypolipidemische Wirkstoffe
Dérivés de l'hydantoine pour l'utilisation comme agents hypoglycémiques et/ou hypolipidémiques

(30) Priority: 23.02.1990 JP 43420/90
(43) Date of publication of application: 04.09.1991
(73) Proprietor: MOCHIDA PHARMACEUTICAL CO., LTD., Shinjuku-ku Tokyo 160 (JP)
(72) Inventor: Mochida, Ei, Toshima-ku, Tokyo 170 (JP); Murakami, Kimihiro, Suntou-gun, Shizuoka 410-14 (JP); Kato, Kazuo, Mishima-shi, Shizuoka 411 (JP); Kato, Katsuaki, Koganei-shi, Tokyo 184 (JP); Okuda, Jun, Tenpaku-ku, Nagoya-shi, Aichi 468 (JP); Miwa, Ichitomo, Tenpaku-ku, Nagoya-shi, Aichi 468 (JP)
(74) Representative: Hansen, Bernd, Dr. Dipl.-Chem.

(56) References cited:
- EP-A- 0 251 784
- EP-A- 0 305 947
- EP-A- 0 355 827
- BIOCHEMICAL PHARMACOLOGY, vol. 40, no. 2, 1990, pages 303-307, Pergamon Press plc, GB; I. MIWA et al.: "In vivo activities of aldose reductase inhibitors having a 1-(arylsulfonyl)hydantoin structure"
- BIOCHEMICAL PHARMACOLOGY, vol. 36, no. 17, 1987, pages 2789-2794, Pergamon Journals, Ltd, GB; I. MIWA et al.: "Development of potent aldose reductase inhibitors having a hydantoin structure"
- CHEM. PHARM. BULL., vol. 37, no. 6, June 1989, pages 1581-1582, Pharmaceutical Society of Japan; I. MIWA et al.: "Improvement of nerve conduction velocity in mutant diabetic mice by aldose reductase inhibitor without affecting nerve myo- inositol content"
- CHEM. PHARM. BULL., vol. 32, no. 5, 1984, pages 2030-2032; I. MIWA et al.: "Hypoglycemic activity of aldose reductase inhibitor, 1-[(p- bromophenyl)sulfonyl]hydantoin"
- PATENT ABSTRACTS OF JAPAN, vol. 7, no. 209 (C-186)[1354], 14th September 1983; & JP-A-58 109 418
- PATENT ABSTRACTS OF JAPAN, vol. 12, no. 56 (C-477)[2903], 19th February 1988; & JP-A-62 201 873
- PATENT ABSTRACTS OF JAPAN, vol. 11, no. 267 (C-443)[2714], 28th August 1987; & JP-A-62 67 075

## Description

The present invention relates to the use of pharmaceutical composition containing at least one of hydantoin derivatives as claimed in claim 1.

In spite of the early discovery of insulin and its subsequent wide - spread use in the treatment of diabetes mellitus, and the later discovery and use of sulfonylureas (e.g. chlorpropamide, tolbutamide) and biguanides (e.g. phenformin) as oral hypoglycemic agents, the treatment of diabetes mellitus remains less than satisfactory. Insulin can only be administered intravenously due to its chemical nature, and therefore, is troublesome and inconvenient to use. Oral hypoglycemic agents tend to promote side effects such as excessive hypoglycemia or lactic acidosis. A continuing need for potent hypoglycemic agents, which may be less toxic, is clearly evident.

Recently, developments of the aldose reductase (AR) inhibitors as agents for diabetic complications are in progress. AR inhibitors will not be agents for diabetes mellitus, but symptomatic agents for diabetic complications, so they are expected to be little effective against diabetes mellitus itself.

Diabetes mellitus should be treated by hypoglycemic agents, and preferably, by the hypoglycemic agents with AR inhibiting activity, so such agents having both hypoglycemic and AR inhibiting activities have been desired.

Further, diabetes mellitus is usually accompanied by cardiovascular disease due to atherosclerosis, so the hypoglycemic agents with hypolipidemic activities have also been desired.

EP-A-0 251 784 discloses hydantoin derivatives comprising a substituted naphthalene group, wherein said naphthalene group comprises four substituents. These substituents are at the positions 4 to 7 of said naphthalene ring. According to one Example a bromine atom is at the position 5 of the naphthalene ring. It is additionally preferred that the linkage to the -SO₂-group is at position 1. The reduction rate of the serum glucose of a known compound of this type is too low and is therefore insufficient.

EP-A-0 355 827, which is a document in accordance with Article 54(3) EPC, teaches some hydantoin derivatives having reducing activities of hypoglycemia in animal models. However, this document remains silent with regard to the treatment of diabetes mellitus with or without hyperlipidemea.

Chem.Pharm.Bull. 32(5) 2030-2032 (1984) reveals the hypoglycemic activity of a specific p-bromophenyl hydantoin derivative. This document does not disclose the hypoglycemic activity in a diseased state of hyperserum glucose which is a rather substantive problem.

Biochemical Pharmacology, Vol. 36, No. 17, 1987 pp. 2789-2794 discloses the hypoglycemic activity of the β-naphthyl sulfonyl hydantoin derivatives. After administration of these compounds to normal rabbits, the blood glucose level was reduced. The blood glucose levels of diabetic rats after the oral administration of these compounds for twelve days were not significantly different from those of the control rats, that is to say the untreated rats.

Accordingly, the compounds of the latter two mentioned documents caused hypoglycemic activities in normal animals and thus produced side-effects of excessive hypoglycemia is used in medicaments. The compositions do not show hypogycemic activity in diabetic diseased animal models.

An object of the present invention is to provide pharmaceutical compositions containing at least one of hydantoin derivatives having a hypoglycemic activity.

The present inventors previously found that sulfonylhydantoin derivatives had strong inhibiting activities against AR and accomplished an invention of AR inhibitors (JP Kokai Nos. Sho 58 109418, Sho 62 67075, Sho 62 201873 and Hei 1 61465). And M. S. Malamas et al. disclosed naphthalenesulfonyl hydantoin derivatives useful as AR inhibitors (U.S. Pat. No. 4,743,611).

Furthermore, the present inventors have made extensive researches on a series of hydantoin derivatives, and have found the hydantoin derivatives of the present invention having extremely strong hypoglycemic activities as well as hypolipidemic activities with lesser side effects. The compounds of the present invention are extremely useful for the treatment and/or prevention of diabetes mellitus.

The present invention relates to the use of a pharmaceutical composition containing at least one of hydantoin derivatives represented by formula (I): 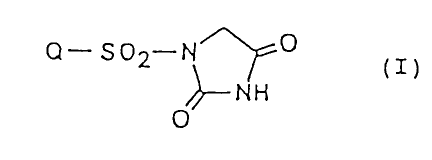 non-toxic salts, solvates or solvates of non-toxic salts thereof; wherein Q represents an alkyl group having 1 to 8 carbon atoms, a cycloalkyl group having 3 to 6 carbon atoms, a biphenylyl group, a mono- or a fused heterocyclic group which may be substituted by one or more substituents which are same or different and selected from a group consisting of a halogen atom, a C₁₋₄ alkyl group, a nitro group, a cyano group, an optionally protected carboxy group, an optionally protected carboxymethyl group, a halo-C₁₋₄ alkyl group, a (C₁₋₄-alkyl)-thio group, a (C₁₋₄ alkyl)-carbonyl group, a C₁₋₄ alkoxy group, a (C₁₋₄ alkyl)-sulfinyl group, a (C₁₋₄ alkyl)-sulfonyl group, an optionally protected hydroxy group, an optionally protected amino group, a carbamoyl group or a phenyl group, or a group: 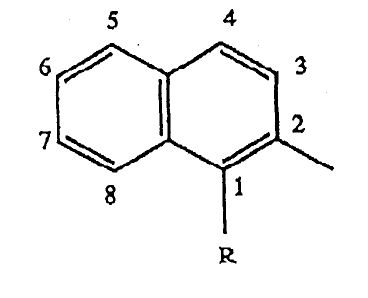 wherein R represents a chlorine or a fluorine atom, with a pharmaceutically acceptable carrier, for preparing a medicament for the treatment and/or prevention of diabetes mellitus with or without hyperlipidemia.

The compounds of the present invention represent hypoglycemic activities and/or hypolipidemic activities in experimental animal models of diabetes mellitus. The compounds of the present invention, however, represent less potent hypoglycemic activities in normal animals. The compounds of the present invention, non - toxic salts, solvates or solvates of non - toxic salts thereof are free of central nervous system side effects such as anticonvulsant activity and little toxic, so useful for the treatment and/or prevention of diabetes mellitus with or without hyperlipidemia. It is indicated that the compounds of the present invention are useful for the treatment and/or prevention of diabetic complications such as somatic or autonomic neuropathy, cataract, retinopathy, nephropathy or microangiopathy. It is also indicated that the compounds of the present invention are useful for the treatment and/or prevention of hyperlipidemia.

In the hydantoin derivatives of the present invention represented by formula (I), it is known that the hydantoin moiety exhibits tautomerism as shown below:

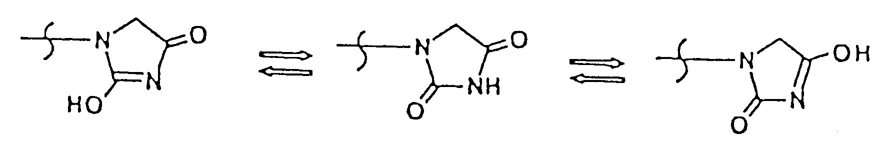

Since these tautomeric isomers are generally deemed to be the same substance, the compounds of the present invention represented by formula (I) also include all of these tautomeric isomers.

The compounds represented by formula (I) may form salts with bases. Typical examples of salts of bases of the compounds represented by formula (I) include pharmaceutically acceptable salts such as alkali metal salts (such as sodium salts, potassium salts, etc.), alkaline earth metal salts (such as magnesium salts, calcium salts, etc.), salts of organic bases (such as ammonium salts, benzylamine salts, diethylamine salts,etc.) or salts of amino acids (such as arginine salts, lysine salts, etc.). These salts of the compounds represented by formula (I) may be mono - salts or di - salts which may be salts of the hydantoin moiety and/or salts of the carboxy group contained in the Q group.

The compounds represented by formula (I) may also form acid addition salts. Typical examples of acid addition salts of the compounds represented by formula (I) include pharmaceutically acceptable salts, such as salts of inorganic acids (such as hydrochlorides, hydrobromides, sulfates, phosphates, etc.), salts of organic acids (such as acetates, citrates, maleates, tartrates, benzoates, ascorbates, ethanesulfonates, toluenesulfonates, etc.) or salts of amino acids (such as aspartates, glutamates, etc.). These salts of the compounds represented by formula (I) may be salts of the heterocyclic moiety in the Q group.

In the compounds of the present invention represented by formula (I), the lower alkyl group can be defined more specifically as a straight or branched lower alkyl group having 1 to 4 carbon atoms such as methyl, ethyl, propyl, isopropyl, butyl, *tert* - butyl, etc. The alkoxy group can be defined more specifically as a straight or branched lower alkoxy group having 1 to 4 carbon atoms such as methoxy, ethoxy, propoxy, isopropoxy, butoxy, *tert* - butoxy, etc. The acyl group can be defined more specifically as a straight or branched lower acyl group having 1 to 5 carbon atoms such as formyl, acetyl, propanoyl, butanoyl, pivaloyl, etc.

In the compounds of the present invention represented by formula (I), the heterocyclic group can be defined as a monocyclic heterocyclic group such as pyrrolyl, pyrrolinyl, imidazolyl, pyrazolyl, triazolyl, tetrazolyl, oxazolyl, isoxazolyl, oxadiazolyl, thiazolyl, isothiazolyl, thiazolinyl, thiadiazolyl, thiatriazolyl, thienyl (thiophenyl), furyl, pyrrolidinyl, imidazolidinyl, thiazolidinyl, pyridyl or its N - oxide, pyrazinyl, pyrimidinyl, pyridazinyl, piperidyl, piperazinyl, morpholinyl, triazinyl, etc., or a fused heterocyclic group such as indolyl, isoindolyl, benzimidazolyl, quinolyl, isoquinolyl, quinazolinyl, cinnolinyl, phthalazinyl, quinoxalinyl, indazolyl, benzotriazolyl, benzoxazolyl, benzoxadiazolyl, benzothiazolyl, benzothiadiazolyl, benzisoxazolyl, benzisothiazolyl, benzothiophenyl (benzo[b]thiophenyl or benzo[c]thiophenyl) (benzothienyl (benzo[b]thienyl or benzo[c]thienyl)), tetrahydrobenzothiophenyl (tetrahydrobenzothienyl), benzofuranyl (benzo[b]furanyl or isobenzofuranyl), chromenyl, chromanyl, coumarinyl, chromonyl, triazolopyridyl, tetrazolopyridyl, purinyl, thiazolopyrimidinyl, triazolopyrimidinyl, thiadiazolopyrimidinyl, thiazolopyridazinyl, naphthyridinyl, xanthenyl, phenoxathiinyl, phenoxazinyl, phenothiazinyl, carbazolyl, etc. preferably indolyl, benzimidazolyl, benzotriazolyl, benzothiazolyl, benzisoxazolyl, benzisothiazolyl, benzothienyl, tetrahydrobenzothienyl, benzofuranyl, coumarinyl, chromonyl, more preferably benzo[b]thienyl or benzo[b]furanyl. The above-mentioned heterocyclic groups may be substituted with a group such as a lower alkyl group (such as methyl, ethyl, propyl, isopropyl, butyl, *tert* - butyl, etc.), a lower alkylcarbonyl group (such as formyl, acetyl, propanoyl, butanoyl, pivaloyl, etc.), a lower alkoxy group (such as methoxy, ethoxy, propoxy, isopropoxy, butoxy, *tert* - butoxy, etc.), a phenyl group, a cyano group, a carbamoyl group, an optionally protected carboxy group, an optionally protected carboxymethyl group, a nitro group, a lower haloalkyl group (such as trifluoromethyl, pentafluoroethyl, etc.), an optionally protected hydroxy group, an optionally protected amino group, (such as acyl amino, etc.), a lower alkylthio group, a lower alkylsulfinyl group, a lower alkyl sulfonyl group or a halogen atom (such as fluorine, chlorine, bromine, iodine, etc.), or combination of any of these groups.

In a mono - heterocyclic group, a compound unsubstituted or substituted with 1 or 2 substituents which are the same or different and selected from a group consisting of a halogen atom or a phenyl group, is preferable.

In a fused heterocyclic group, a compound unsubstituted or substituted with 1 to 3 substituents which are the same or different and selected from a group consisting of a halogen atom, a lower alkyl group, a lower haloalkyl group, a lower alkylthio group or a cyano group, is preferable.

When a fused heterocyclic group is a benzo[b]furan-2 - yl group which may be substituted, the said substituents are preferably 1 to 3 halogen atoms.

The compounds of the present invention represented by formula (I) can be produced by the processes described in JP Kokai No. Hei 1 61465 or JPA No. Hei 1 217697.

To demonstrate the utility of the compounds of the present invention, experimental examples of representative compounds are shown below.

### Compounds in the present invention

- Compound 1:: 1-(1-chloronaphthalen-2-ylsulfonyl)hydantoin
- Compound 2:: 1-(3,6-dichloronaphthalen-2-ylsulfonyl)hydantoin
- Compound 3:: 1-(5-chloronaphthalen-2-ylsulfonyl)hydantoin
- Compound 4:: 1-(6-methyl-5-nitronaphthalen-2-ylsulfonyl)hydantoin
- Compound 5:: 1-(7-methylnaphthalen-2-ylsulfonyl)hydantoin
- Compound 6:: 1-(6-methoxy-5-nitronaphthalen-2-ylsulfonyl)hydantoin
- Compound 7:: 1-(1-fluoronaphthalen-2-ylsulfonyl)hydantoin
- Compound 8:: 1-(benzo[b]thien-2-ylsulfonyl)hydantoin
- Compound 9:: 1-(benzo[b]furan-2-ylsulfonyl)hydantoin
- Compound 10:: 1-(5-fluorobenzo[b]thien-2-ylsulfonyl)hydantoin
- Compound 11:: 1-(3-chlorobenzo[b]thien-2-ylsulfonyl)hydantoin
- Compound 12:: 1-(4-chlorobenzo[b]thien-2-ylsulfonyl)hydantoin
- Compound 13:: 1-(5-bromobenzo[b]furan-2-ylsulfonyl)hydantoin
- Compound 14:: 1-(5-chlorobenzo[b]furan-2-ylsulfonyl)hydantoin
- Compound 15:: 1-(benzo[b]isothiazol-3-ylsulfonyl)hydantoin
- Compound 16:: 1-(2,5-dichlorothien-3-ylsulfonyl)hydantoin
- Compound 17:: 1-(benzothiazol-2-ylsulfonyl)hydantoin
- Compound 18:: 1-(6-chlorobenzo[b]thien-2-ylsulfonyl)hydantoin
- Compound 19:: 1-(4,6-dichlorobenzo[b]furan-2-ylsulfonyl)hydantoin
- Compound 20:: 1-(3-bromobenzo[b]furan-2-ylsulfonyl)hydantoin
- Compound 21:: 1-(3-bromo-7-fluorobenzo[b]thien-2-ylsulfonyl)hydantoin
- Compound 22:: 1-(2-chlorobenzo[b]thien-3-ylsulfonyl)hydantoin
- Compound 23:: 1-(3-chlorobenzo[b]furan-2-ylsulfonyl)hydantoin
- Compound 24:: 1-(4-bromobenzo[b]furan-2-ylsulfonyl)hydantoin
- Compound 25:: 1-(7-fluorobenzo[b]furan-2-ylsulfonyl)hydantoin
- Compound 26:: 1-(4,5-dichlorobenzo[b]furan-2-ylsulfonyl)hydantoin
- Compound 27:: 1-(5,6-dichlorobenzo[b]furan-2-ylsulfonyl)hydantoin
- Compound 28:: 1-(3-bromo-7-fluorobenzo[b]furan-2-ylsulfonyl)hydantoin
- Compound 29:: 1-(6-bromobenzo[b]furan-2-ylsulfonyl)hydantoin
- Compound 30:: 1-(3-iodobenzo[b]furan-2-ylsulfonyl)hydantoin
- Compound 31:: 1-(3,6-dibromobenzo[b]furan-2-ylsulfonyl)hydantoin
- Compound 32:: 1-(3-bromonaphthalen-2-ylsulfonyl)hydantoin

### Reference compound

- Compound A:: gliclazide (Glimicron ® ; manufactured by Dainippon Pharm. Co. LTD.)

### Experimental Example 1

### Hypoglycemic effects in streptozotocin-diabetic rats

Diabetic rats were produced according to the method of Junod *et al*. (A. Junod *et al*., J. Clin. Invest., 48, 2129 (1969)). Briefly, male Wistar rats were given 26.5 mg/kg of streptozotocin (STZ) intravenously. Four days later, the rats with serum glucose levels of between 400 and 500 mg/dl were used. After being fasted for 4 hrs, groups of 5 diabetic rats were orally administered with the compounds of the present invention or gliclazide in a dose of 100 mg/kg. Blood samples were collected from the orbital vein before and 6 hrs after the administration. Serum glucose levels were determined by the enzymatic methods with an autoanalyzer (Cobas Fara ® , Roche). The results are shown in Table 1.

**Table 1**

| Compounds | Lowering rate of serum glucose (%) |
|---|---|
| 1 | 37.2 |
| 2 | 23.2 |
| 3 | 19.0 |
| 7 | 48.8 |
| 8 | 33.5 |
| 9 | 52.1 |
| 10 | 16.0 |
| 12 | 11.0 |
| 13 | 22.3 |
| 14 | 12.8 |
| 15 | 23.6 |
| 17 | 15.0 |
| 18 | 33.1 |
| 19 | 14.5 |
| 20 | 33.5 |
| 21 | 13.2 |
| 22 | 14.0 |
| 23 | 44.7 |
| 24 | 11.0 |
| 25 | 45.9 |
| 26 | 21.1 |
| 27 | 28.5 |
| 28 | 49.2 |
| 29 | 29.0 |
| 30 | 19.0 |
| 31 | 29.0 |
| 32 | 17.4 |
| A | 11.0 |

All compounds of the present invention listed in table 1 showed significant hypoglycemic activities.

### Experimental Example 2

### Ketone body lowering effects in streptozotocin-diabetic rats

Diabetic rats were produced according to the method described previously. Groups of 5 diabetic rats were orally administered with compound 20 or 23 of the present invention for 2 weeks. The rats were deprived of food 4 hrs before the last administration, and 6 hrs after the last administration, blood samples were collected from the orbital vein. Serum total ketone body (KB) levels were determined by the enzymatic method with an autoanalyzer (Cobas Fara ® , Roche). In this experiment, serum total ketone body levels in streptozotocin - diabetic rats increased by 92 % of normal rats in the mean value. The results are shown in table 2.

**Table 2**

| Compounds | Dose (mg/kg) | Lowering rate of serum total ketone body (%) |
|---|---|---|
| 20 | 100 | 50 |
| 23 | 100 | 43 |

Compounds 20 and 23 of the present invention showed lowering activity of serum total ketone body levels.

### Experimental Example 3

### Hypoglycemic effects in normal rats

Groups of 5 male Wistar rats, after being fasted for 4 hrs, were orally administered with compound 20 or 23 of the present invention or gliclazide. Blood samples were collected from the orbital vein before, 1 hr and 6 hrs after the administration. Serum glucose levels were determined by the enzymatic method with an autoanalyser (Cobas Fara ® , Roche). The results are shown in table 3.

**Table 3**

| Compounds | Dose (mg/kg, p.o.) | Lowering rate of serum glucose (%) | |
|---|---|---|---|
| | | Time after administration (hour) | |
| | | 1 | 6 |
| 20 | 100 | 13 | 6 |
| 23 | 100 | 9 | 0 |
| A | 10 | 57 | 1 |

Compounds 20 and 23 of the present invention showed lesser influences on serum glucose level of normal rats. On the other hand, gliclazide (compound A) significantly lowered serum glucose level of normal rats.

### Experimental Example 4

### Hypolipidemic effects in streptozotocin - diabetic rats

Diabetic rats were produced according to the method described previously. Groups of 5 diabetic rats were orally administered with compound 20 or 23 of the present invention at a dose of 100 mg/kg for 2 weeks. The rats were deprived of food 4 hrs before the last administration, and 6 hrs after the last administration, blood samples were collected from the orbital vein. Serum triglyceride (TG), non - esterified fatty acid (NEFA) and cholesterol (CHOL) levels were determined by the enzymatic method with an autoanalyzer (Cobas Fara ® , Roche). In this experiment, each parameters of serum lipids in streptozotocin - diabetic rats increased by 40 % of normal rats in the mean value. The results are shown in table 4.

**Table 4**

| Compounds | Dose mg/kg | Lowering rate of serum lipids (%) | | |
|---|---|---|---|---|
| | | TG | NEFA | CHOL |
| 20 | 100 | 36 | 35 | 31 |
| 23 | 100 | 43 | 18 | 30 |

Compounds 20 and 23 of the present invention showed lowering activities of serum triglyceride, non - esterified fatty acid and cholesterol levels.

### Experimental Example 5

Hydantoin derivatives of the present invention were examined for acute toxicity. Groups of 5 ICR strain mice were orally administered with compound 2, 4 to 6, 8, 9, 11 to 14, 16, 20, 23, 25 to 28 of the present invention in a dose of 1 g/kg, and no change was observed in any of the groups over the one - week period after the administration.

The compounds of the present invention, non - toxic salts, solvates or non - toxic salts thereof showed significant hypoglycemic activities and/or hypolipidemic activities in experimental animal models of diabetes mellitus. The compounds of the present invention, however, showed less potent hypoglycemic activities in normal animals. On the other hand, gliclazide, a reference compound, showed less potent hypoglycemic activity than all compounds of the present invention in experimental animal model of diabetic mellitus, while it showed remarkable hypoglycemic activity in normal animals. Furthermore, the compounds of the present invention showed little toxicities. The results indicate that the compounds of the present invention are useful for the treatment and/or prevention of diabetes mellitus with or without hyperlipidemia. It is also indicated that the compounds of the present invention are useful for the treatment and/or prevention of diabetic complications such as somatic or autonomic neuropathy, cataract, retinopathy, nephropathy or microangiopathy. It is also indicated that the compounds of the present invention are useful for the treatment and/or prevention of hyperlipidemia.

The hydantoin derivatives of the present invention can be employed as pharmaceutical compositions, for example, in the form of pharmaceutical compositions containing hydantoin derivatives together with appropriate pharmaceutically acceptable carrier or medium such as sterilized water, edible oils, pharmaceutically acceptable solvents or solubilizer such as glycerin or propylene glycol. They may be mixed with excipients, binders, lubricants, coloring agents, flavors, emulsifying agents or suspending agents such as Tween 80 or arabic gum to prepare tablets, capsules, powders, granules, subtilized granules, syrups, eye drops, suppositories, ointments, inhalants, aqueous or oily solutions or emulsion or suspensions for injections. These agents can be administered either orally or parenterally (such as intravenous administration, intramuscular administration, subcutaneous administration, intrarectal administration, percutaneous administration or permucosal administration, etc.), and the amount of administration may be in the range of 1 to 5000 mg/day, preferably 10 to 500 mg/day when the preparation is tablets, capsules, powders, injections, suppositories, syrups, inharants or ointments, and may also be adjusted according to the patient conditions and can be administered once or divided 2 to 6 times or by instillation, etc.

The physical properties of the compounds of the present invention which can be prepared by the method described in JP Kokai No. Hei 1 61465 and JPA No. Hei 1 217697 are summarized in the following table 5, but the present invention is not deemed to be limited thereof.

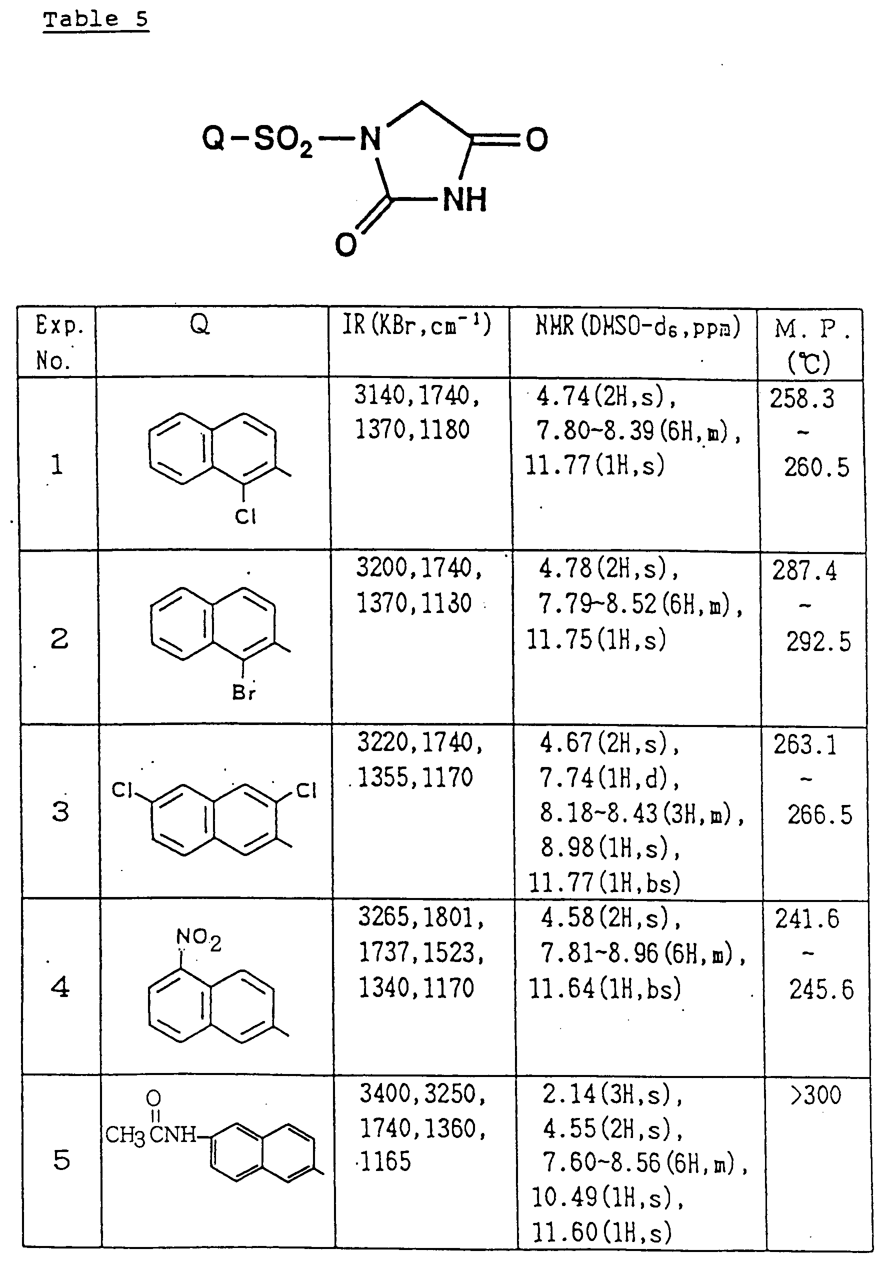

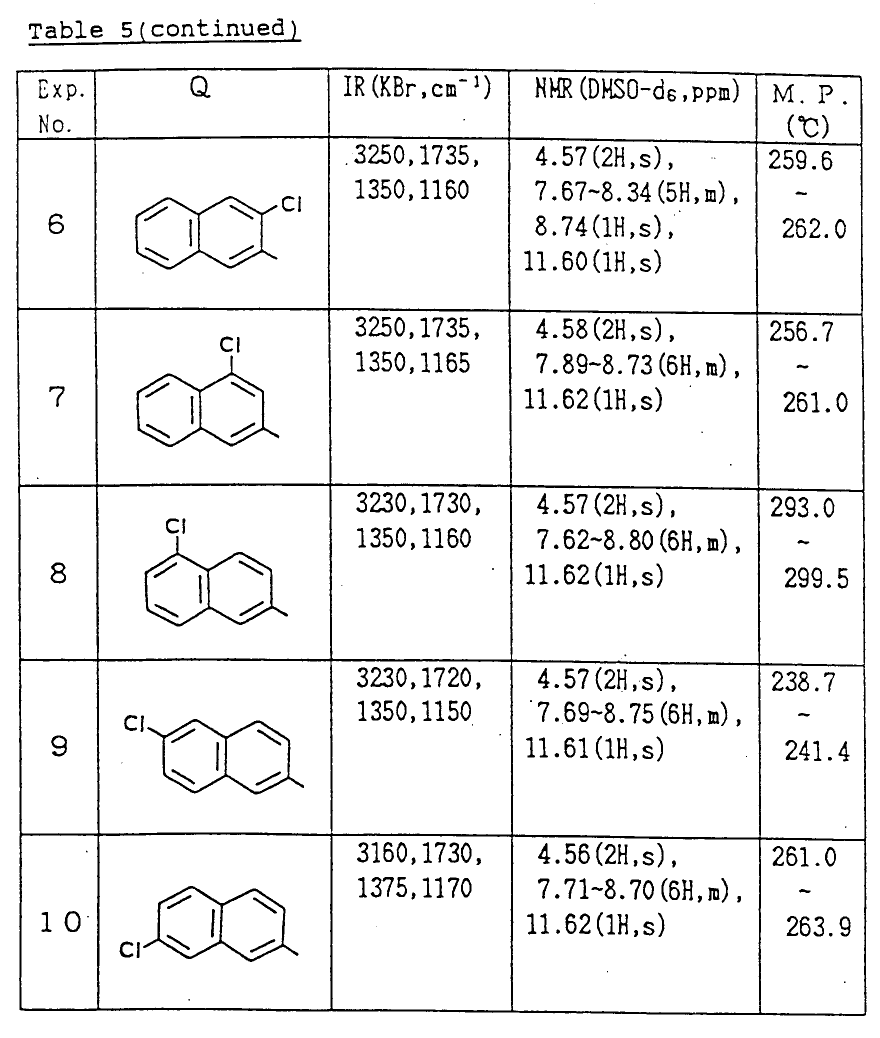

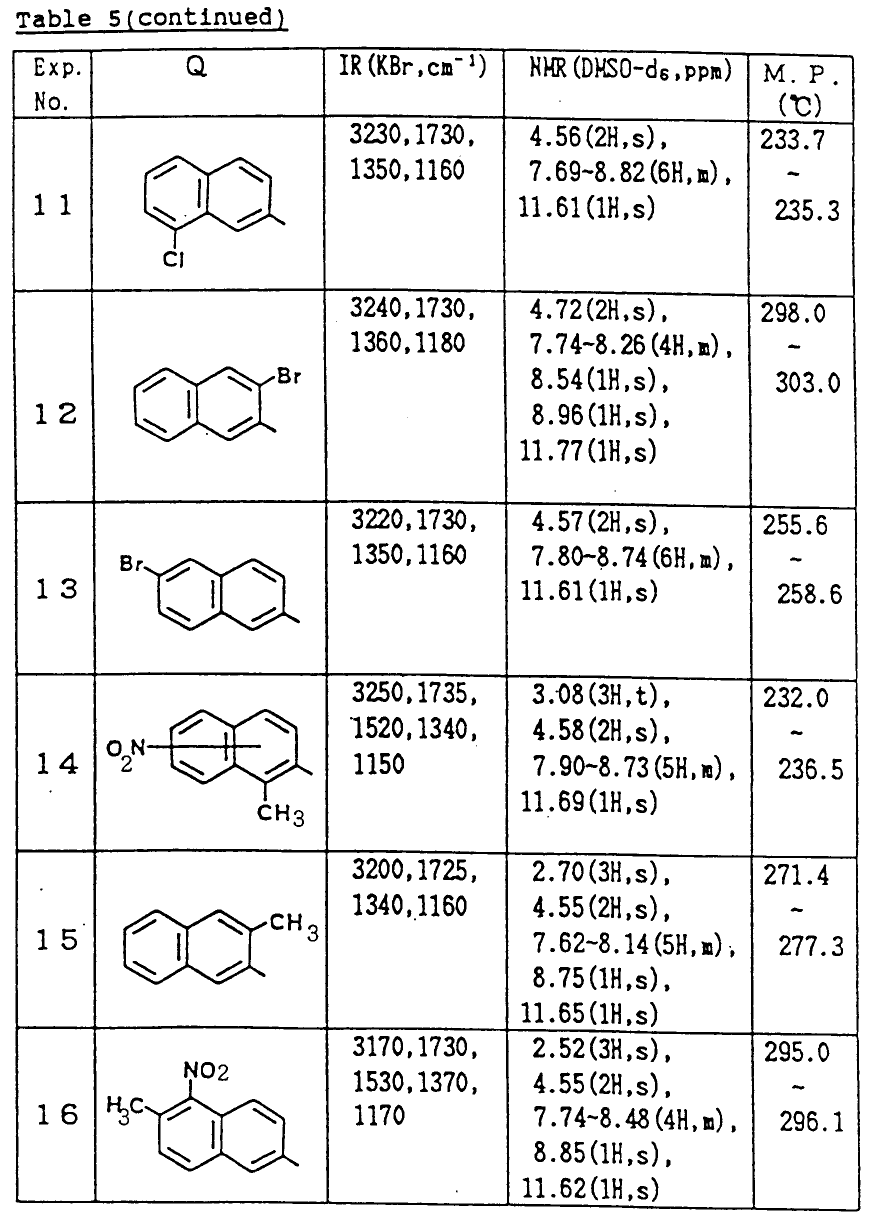

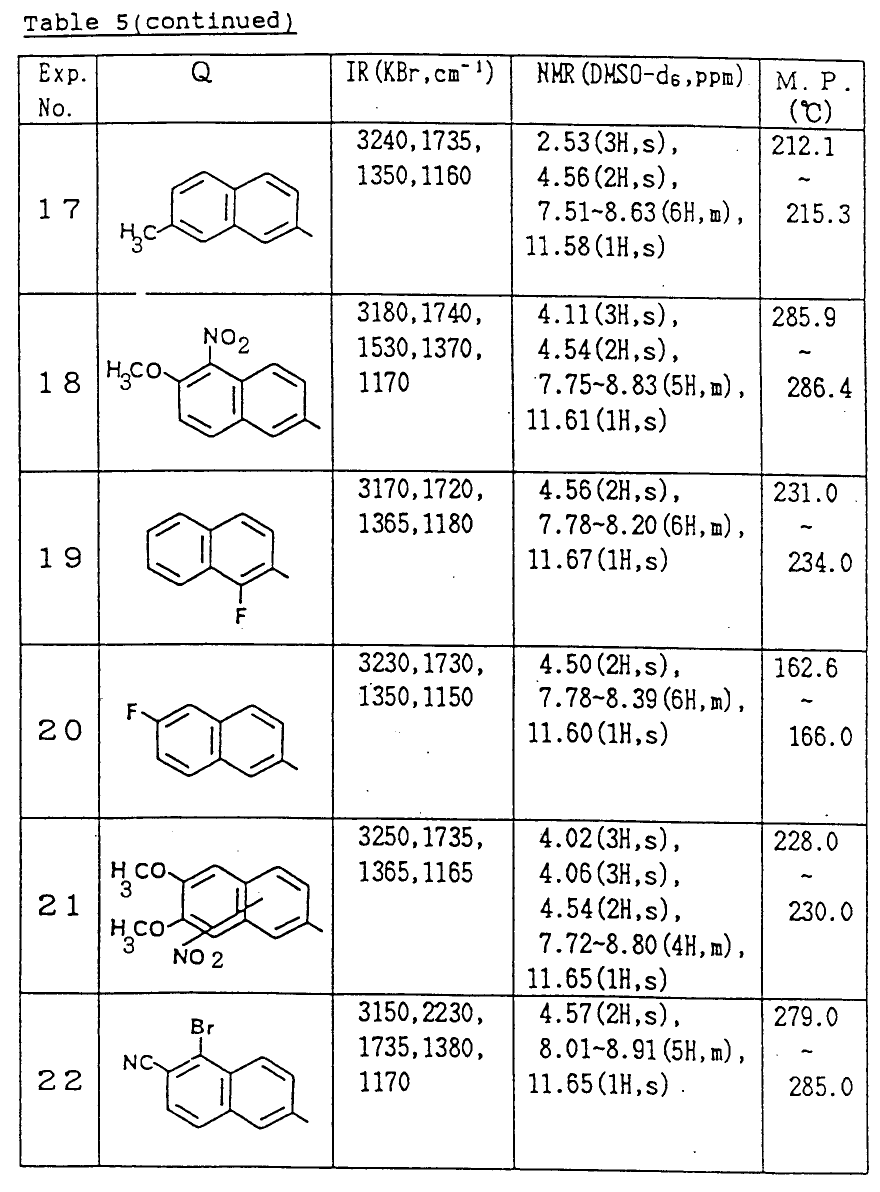

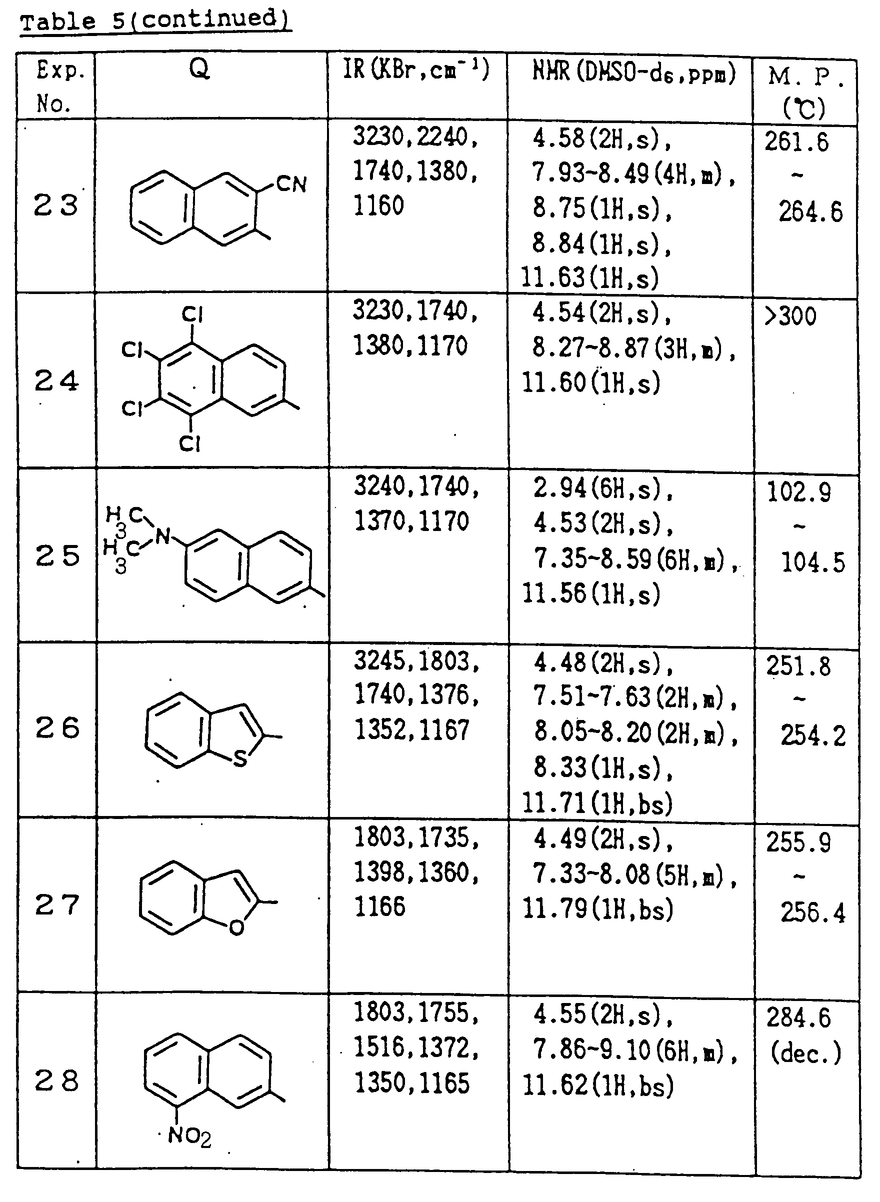

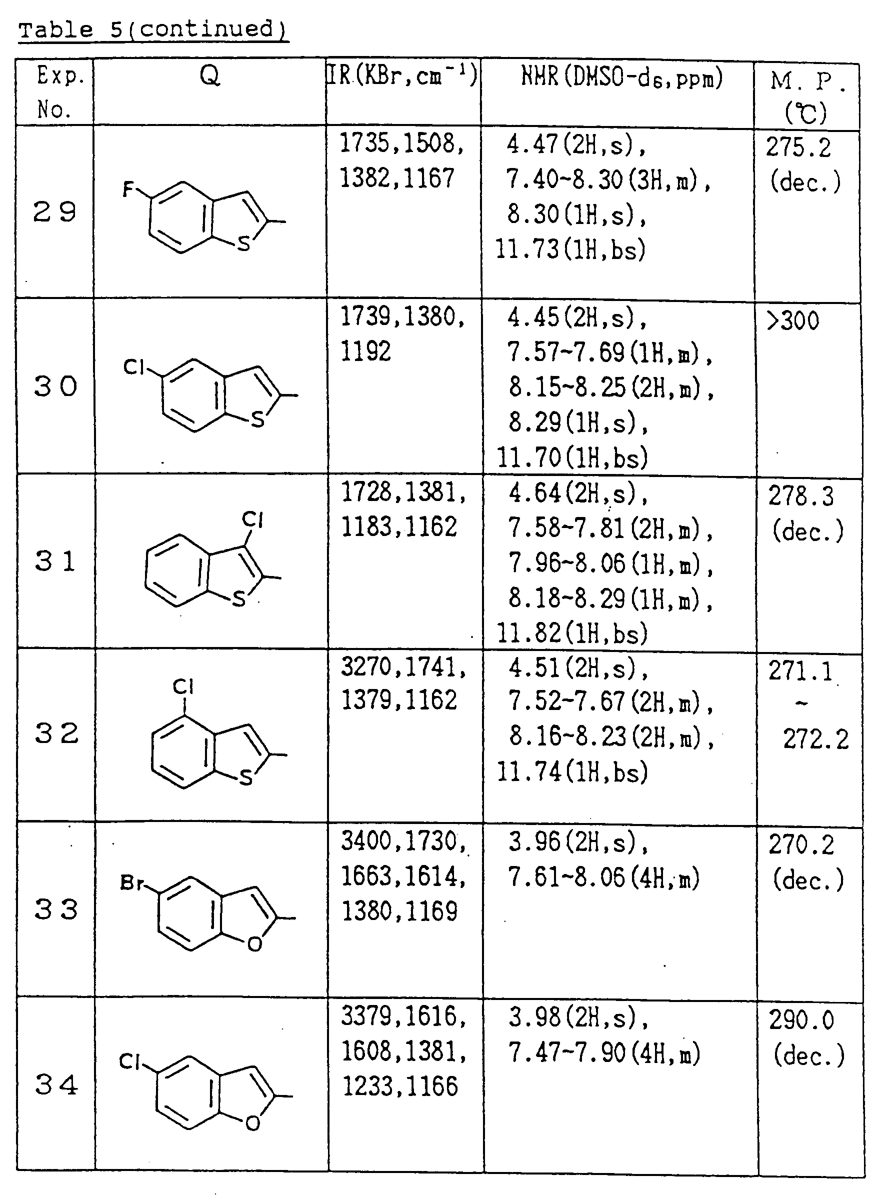

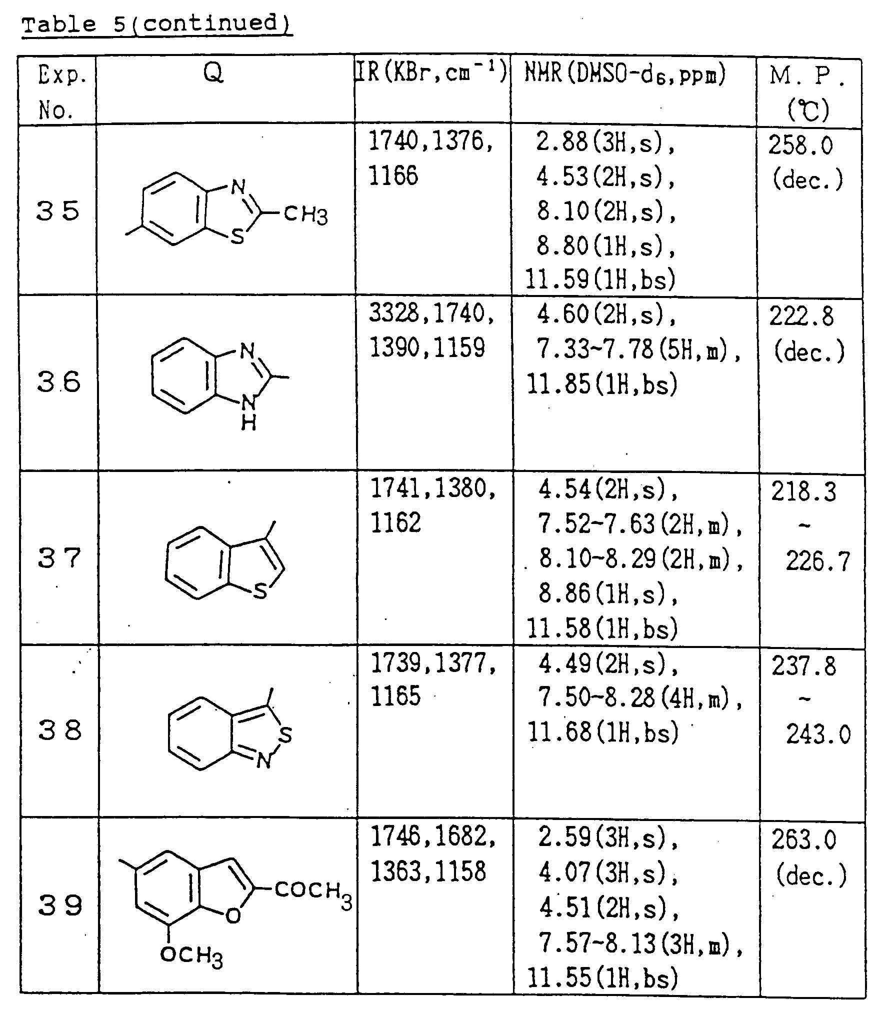

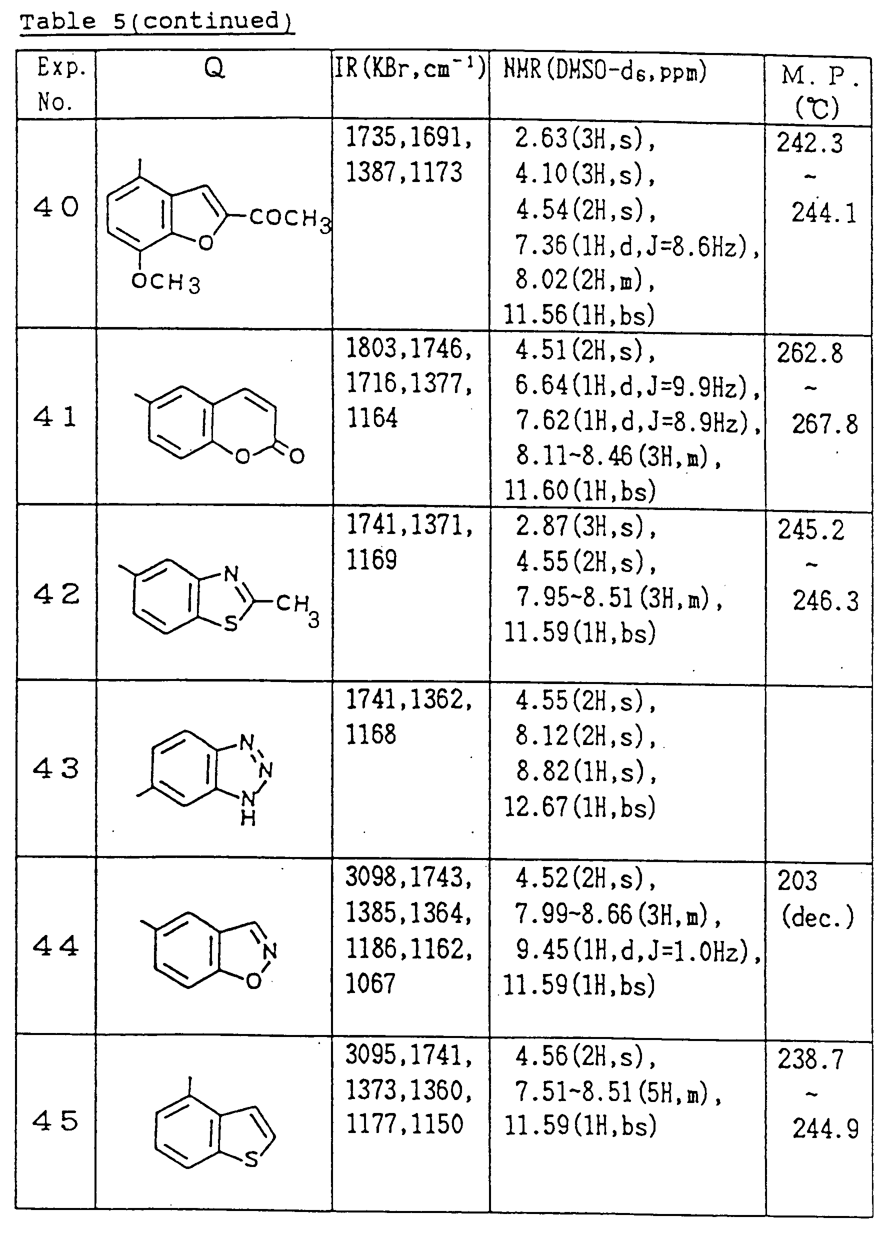

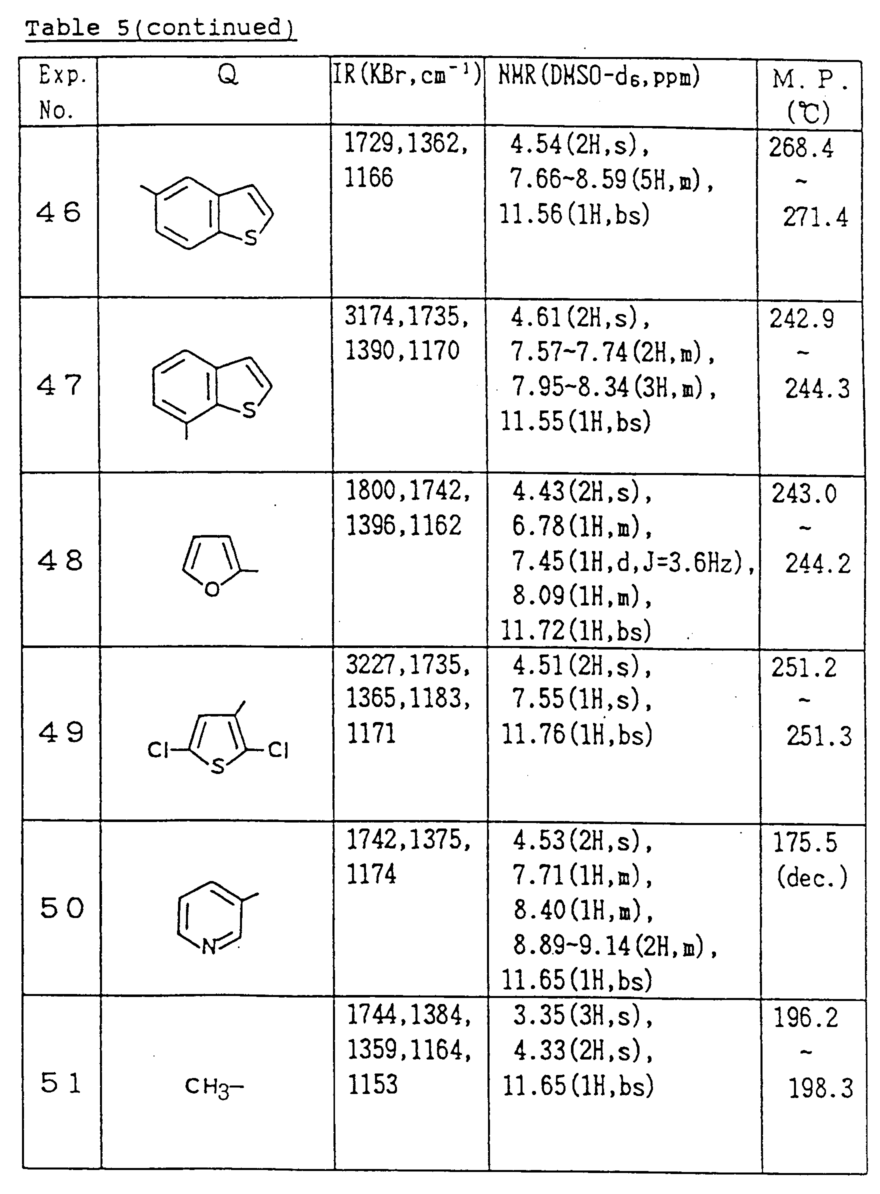

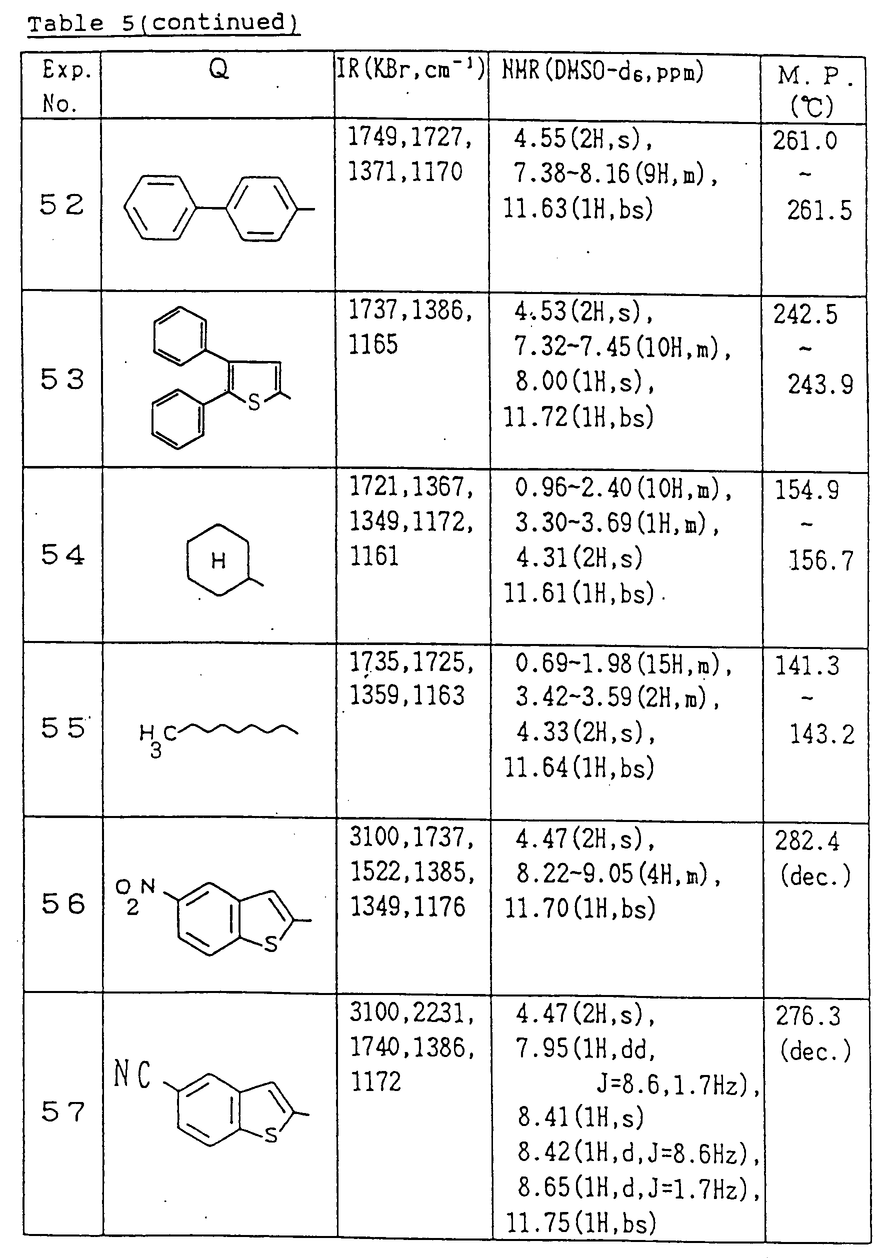

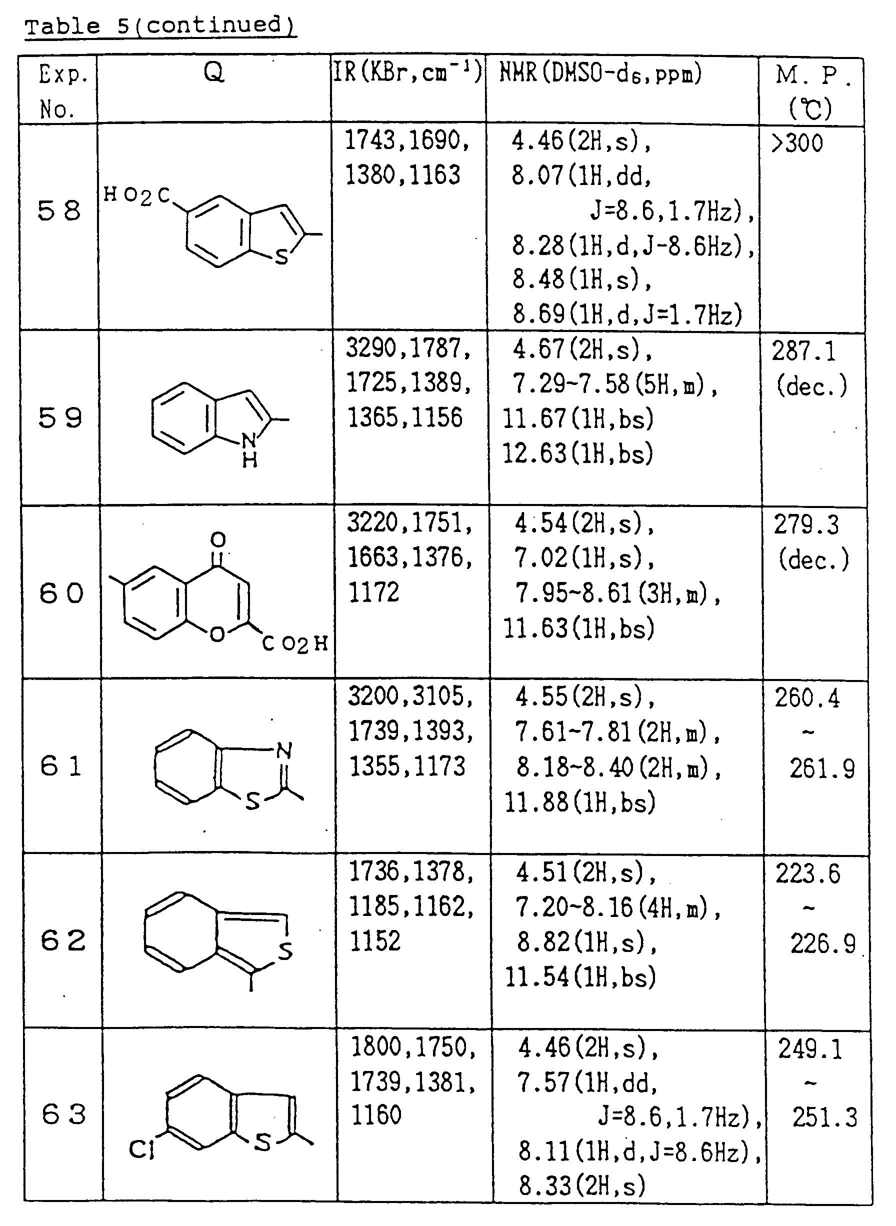

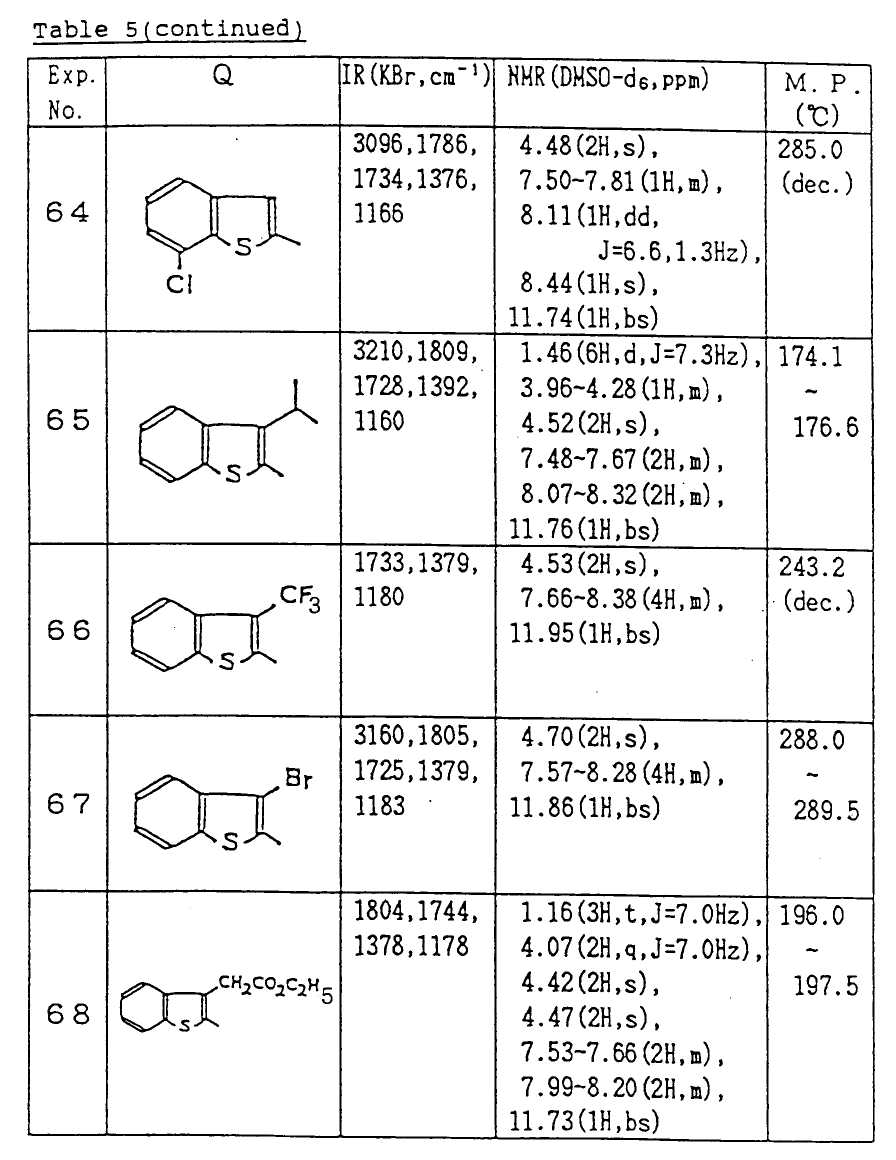

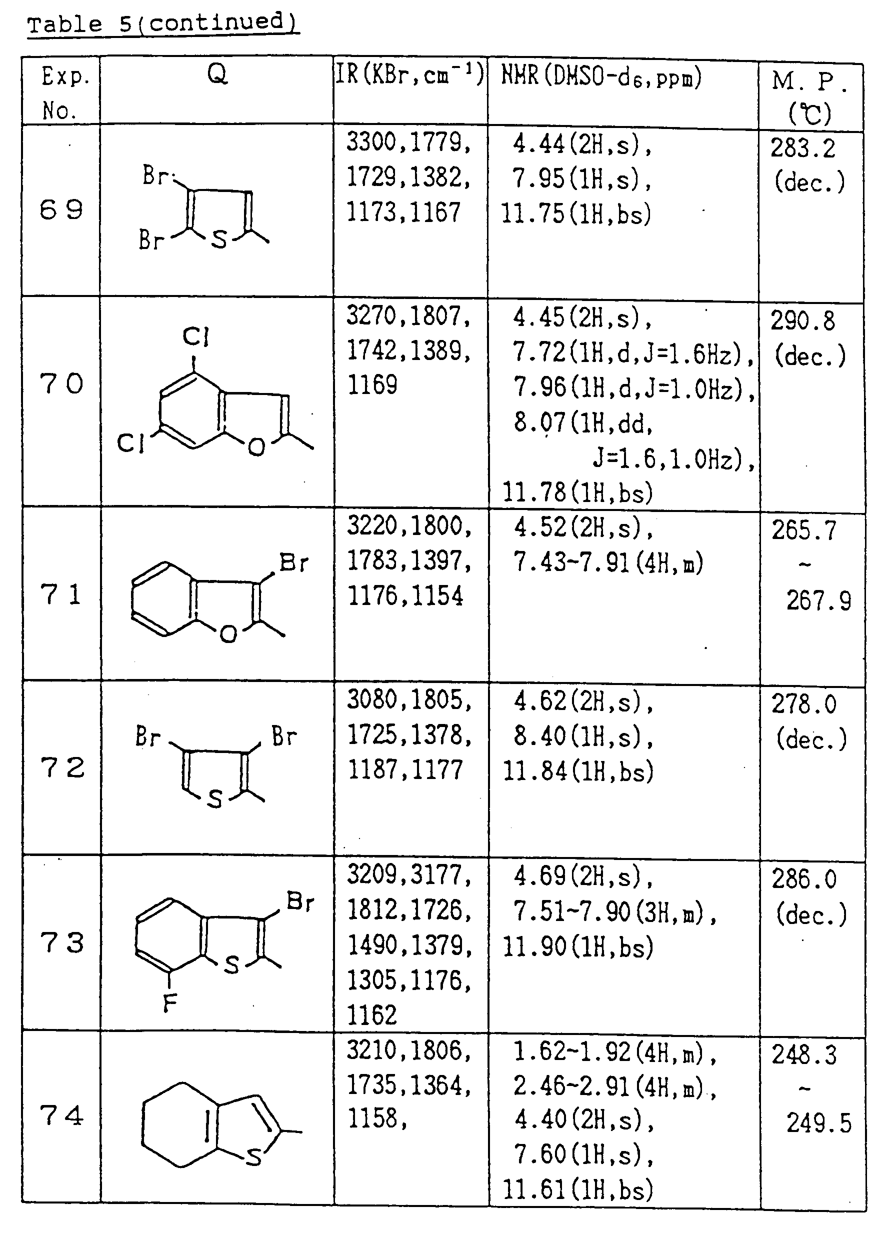

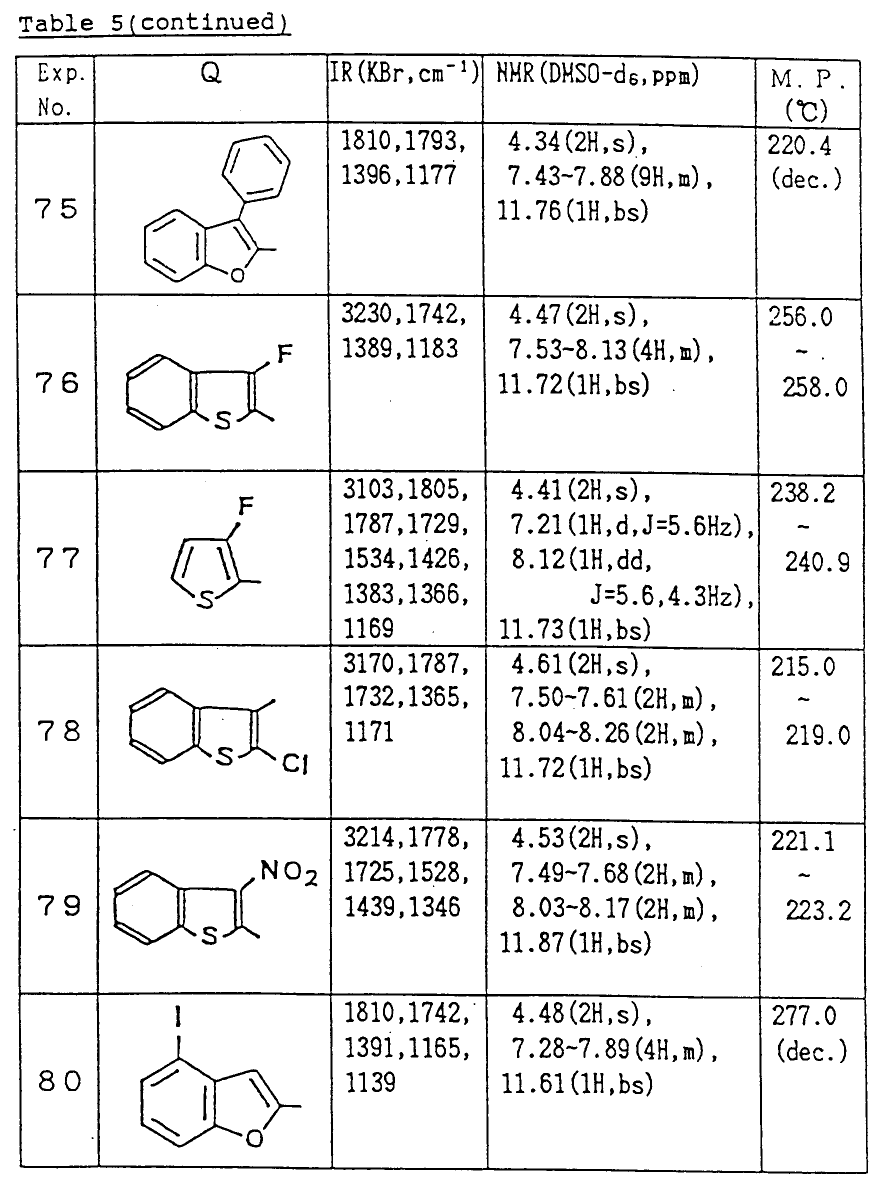

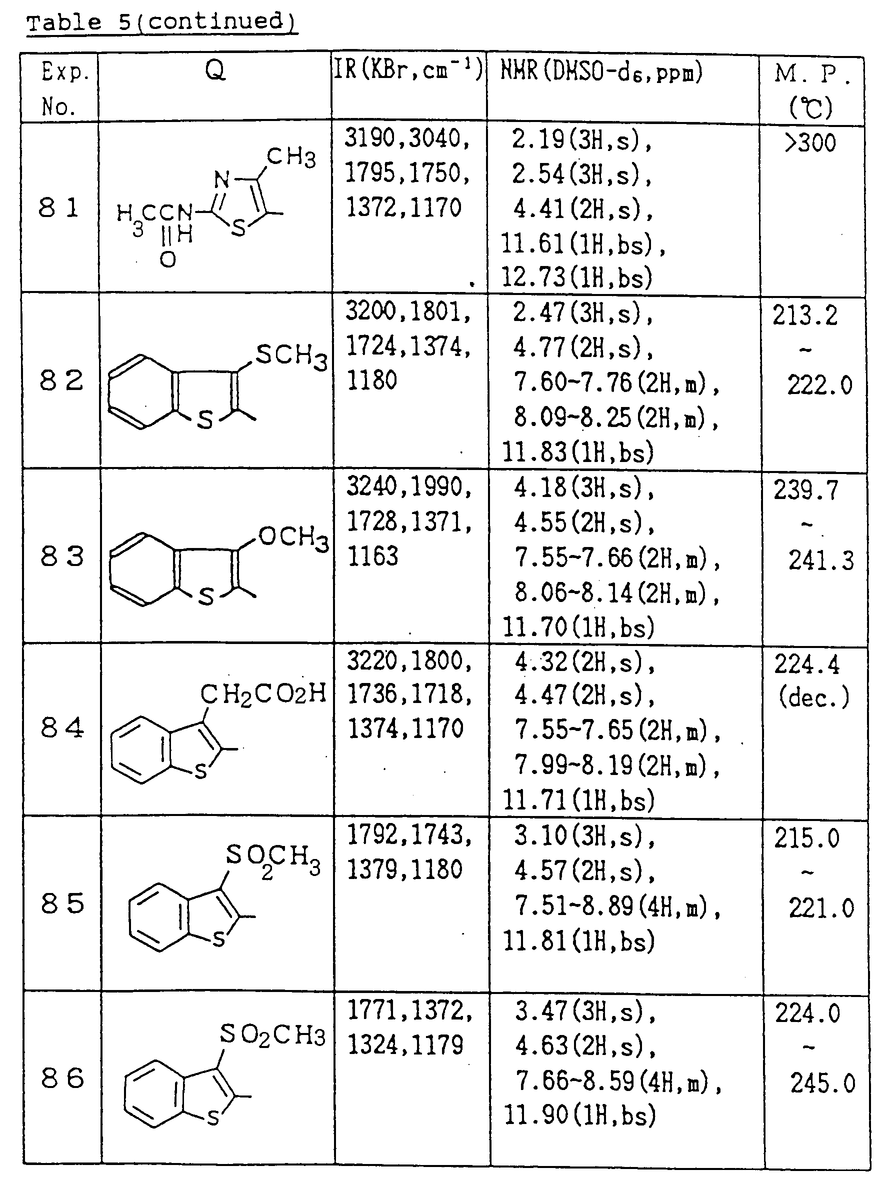

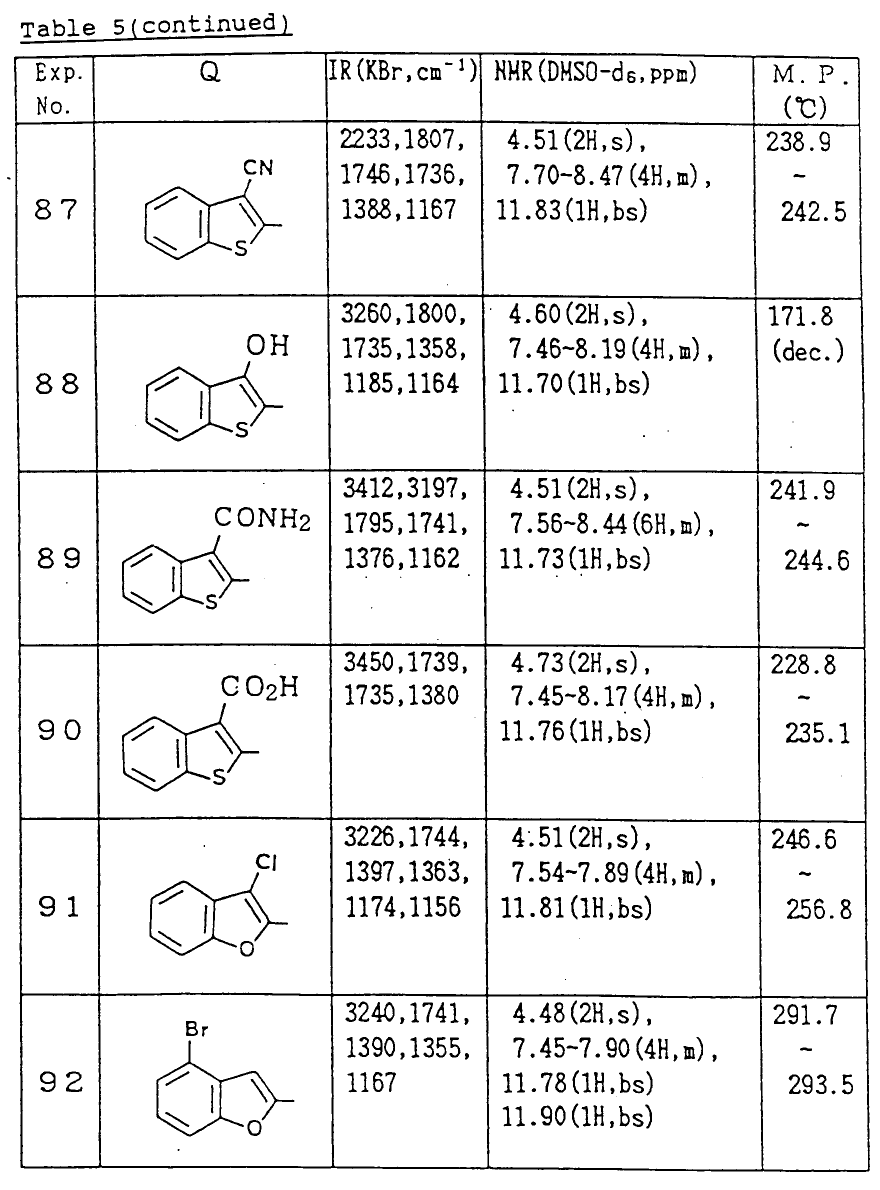

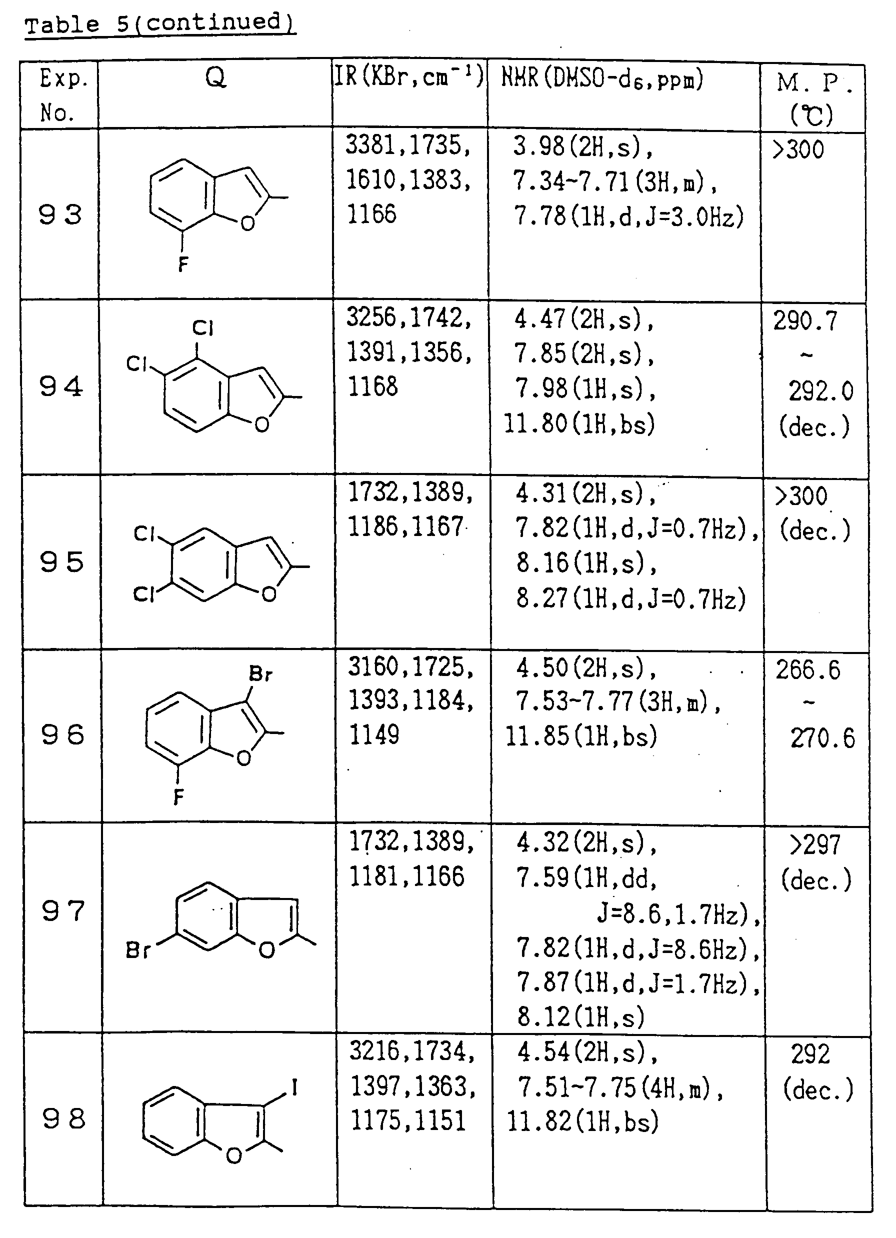

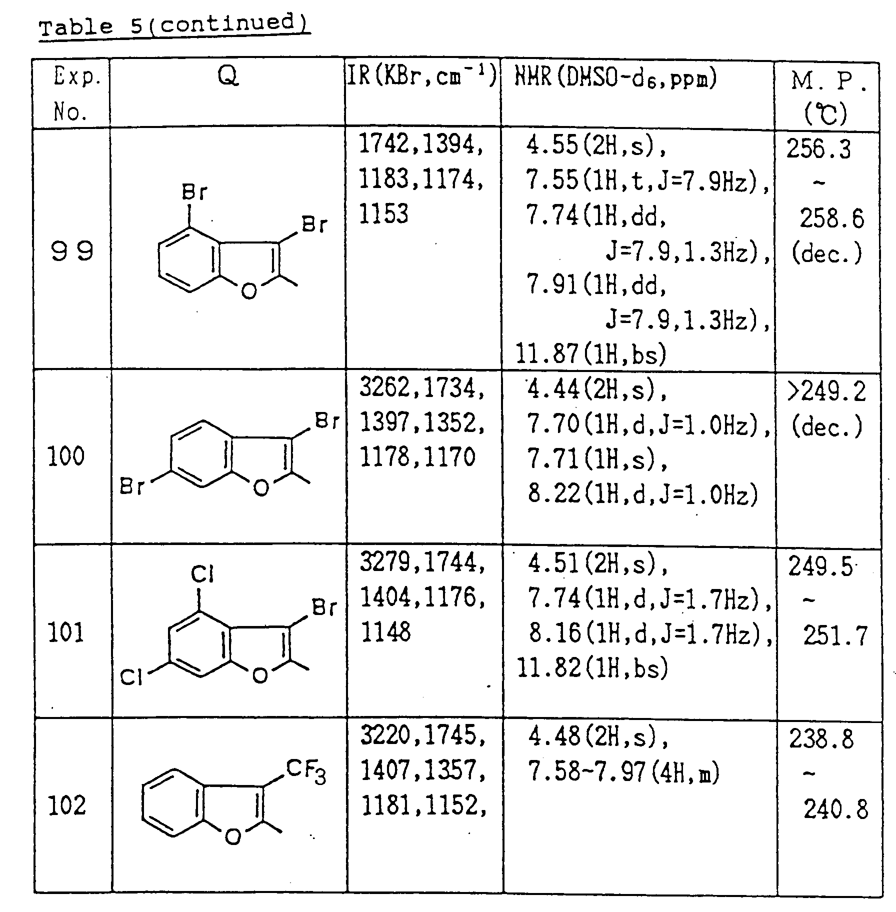

Now, typical but non - limiting examples of formulations of the compound of this invention will be shown below.

### Formulation A (Capsules)

Compound 25, 300 g of weight, 685 g of lactose and 15 g of magnesium stearate were weighed and mixed until the mixture became homogeneous. The mixture was then filled in No. 1 hard gelatin capsule at 200 mg each to obtain capsule preparation.

### Formulation B (Capsules)

Compound 20, 250 g of weight, 730 g of lactose and 20 g of magnesium stearate were weighed and mixed until the mixture became homogeneous. The mixture was then filled in No. 1 hard gelatin capsule at 200 mg each to obtain capsule preparation.

### Formulation C (Tablets)

Compound 21, 300 g of weight, 550 g of lactose, 120 g of potato starch, 15 g of polyvinyl alcohol and 15 g of magnesium stearate were weighed. The weighed amount of compound 21, lactose and potato starch were mixed until accomplishing homogeneity. Then aqueous solution of polyvinylalcohol was added to the mixture and granulated by wet process. The granules were then dried, mixed with magnesium stearate and pressed into tablets, each weighing 200 mg.

### Formulation D (Tablets)

Compound 23, 250 g of weight, 600 g of lactose, 120 g of potato starch, 15 g of polyvinyl alcohol and 15 g of magnesium stearate were weighed. The weighed amount of compound 23, lactose and potato starch were mixed until accomplishing homogeneity. Then aqueous solution of polyvinylalcohol was added to the mixture and granulated by wet process. The granules were then dried, mixed with magnesium stearate and pressed into tablets, each weighing 400 mg.

### Formulation E (Powder)

Compound 7, 200 g of weight, 790 g of lactose and 10 g of magnesium stearate were weighed and mixed until the mixture became homogeneous to obtain 20 % powder preparation.

### Formulation F (Suppositories)

Compound 8, 100 g of weight were weighed and ground by a mortar until the compound became fine powder. Then 180 g of polyethylene glycol 1500 and 720 g of polyethylene glycol 4000 were added to the compound and melted. The mixture was then pressed at 1 g each to obtain suppository preparation.

## Claims

1. Use of a pharmaceutical composition comprising at least one of hydantoin derivatives represented by formula (I): 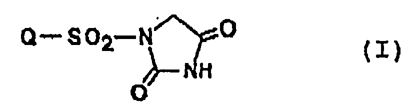 non - toxic salts, solvates or solvates of non - toxic salts thereof; wherein Q represents an alkyl group having 1 to 8 carbon atoms, a cycloalkyl group having 3 to 6 carbon atoms, a biphenylyl group, a monocyclic heterocyclic group or a fused heterocyclic group which may be substituted by one or more substituents which are same or different and selected from a group consisting of a halogen atom, an alkyl group having 1 to 4 carbon atoms, a nitro group, a cyano group, an optionally protected carboxy group, an optionally protected carboxymethyl group, a haloalkyl group having 1 to 4 carbon atoms, an alkylthio group having 1 to 4 carbon atoms, an alkylcarbonyl group wherein alkyl has 1 to 4 carbon atoms, an alkoxy group having 1 to 4 carbon atoms, an alkylsulfinyl group having 1 to 4 carbon atoms, an alkylsulfonyl group having 1 to 4 carbon atoms, an optionally protected hydroxy group, an optionally protected amino group, a carbamoyl group or a phenyl group,
or a group: 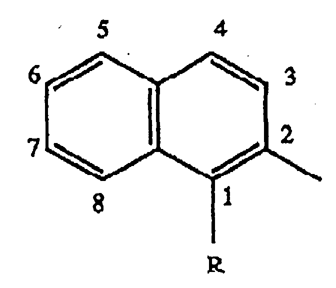 wherein R represents a chlorine or a fluorine atom, with a pharmaceutically acceptable carrier, for preparing a medicament for the treatment and/or prevention of diabetes mellitus with or without hyperlipidemia.

2. Use of a pharmaceutical composition comprising at least one of hydantoin derivatives as defined in claim 1 for the treatment and/or prevention of hyperlipidemia.

3. Use of a pharmaceutical composition as claimed in claim 1 or 2 wherein Q represents a group: 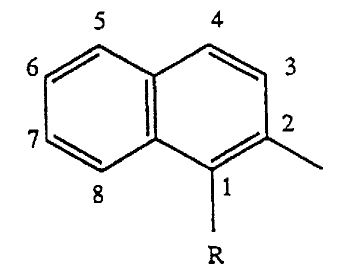 wherein R has the same significance as defined in claim 1.

4. Use of a pharmaceutical composition as claimed in claim 1 or 2 wherein Q represents a benzo[b]thien-2-yl group which may be substituted.

5. Use of a pharmaceutical composition as claimed in claim 1 or 2 wherein Q represents a benzo[b]furan-2-yl group which may be substituted.

6. Use of a pharmaceutical composition as claimed in claim 5 wherein the said substituents are 1 to 3 halogen atoms.

7. Use of hydantoin derivatives represented by formula (I): 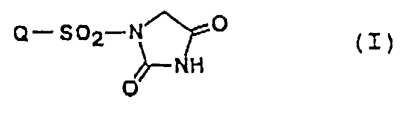 non - toxic salts, solvates or solvates of non - toxic salts thereof; wherein Q has the same significance as defined in claim 1 for the manufacture of a medical preparation or medicament for therapeutic treatment to reduce hyper lipid and/or hyper glucose levels in blood.

## Patentansprüche

1. Verwendung einer pharmazeutischen Zusammensetzung mit einem Gehalt an mindestens einem Hydantoinderivat der Formel (I) 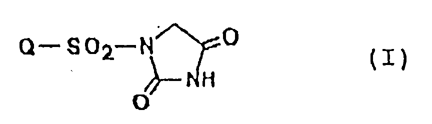 oder von nicht-toxischen Salzen, Solvaten oder Solvaten von nicht-toxischen Salzen davon; wobei Q eine Alkylgruppe mit 1 bis 8 Kohlenstoffatomen, eine Cycloalkylgruppe mit 3 bis 6 Kohlenstoffatomen, eine Biphenylylgruppe, eine monoheterocyclische oder kondensierte heterocyclische Gruppe bedeutet, die durch einen oder mehrere, gleiche oder verschiedene Substituenten substituiert sein kann, die aus folgender Gruppe ausgewählt sind: Halogenatom, C₁₋₄-Alkylgruppe, Nitrogruppe, Cyanogruppe, ggf. geschützte Carboxylgruppe, ggf. geschützte Carboxymethylgruppe, Halogen-C₁₋₄-alkylgruppe, (C₁₋₄-Alkyl)-thiogruppe (C₁₋₄-Alkyl)-carbonylgruppe, C₁₋₄-Alkoxygruppe, (C₁₋₄-Alkyl)-sulfinylgruppe, (C₁₋₄-Alkyl)-sulfonylgruppe, ggf. geschützte Hydroxylgruppe, ggf. geschützte Aminogruppe, Carbamoylgruppe oder Phenylgruppe oder die Gruppe 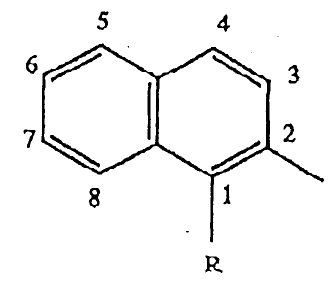 worin R ein Chlor- oder Fluoratom bedeutet, zusammen mit einem pharmazeutisch verträglichen Träger zur Herstellung eines Arzneimittels zur Therapie und/oder Prophylaxe von Diabetes mellitus mit oder ohne Hyperlipidämie.

2. Verwendung einer pharmazeutischen Zusammensetzung, enthaltend mindestens ein Hydantoinderivat nach Anspruch 1 zur Therapie und/oder Prophylaxe von Hyperlipidämie.

3. Verwendung einer pharmazeutischen Zusammensetzung nach Anspruch 1 oder 2, wobei Q die folgende Gruppe bedeutet 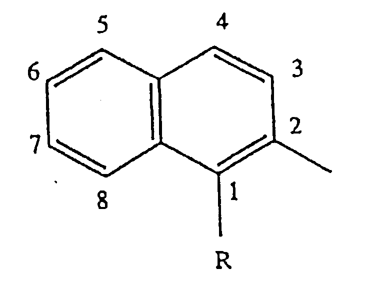 worin R die gleiche Bedeutung wie in Anspruch 1 haben.

4. Verwendung einer pharmazeutischen Zusammensetzung nach Anspruch 1 oder 2, wobei Q eine ggf. substituierte Benzo[b]thien-2-ylgruppe bedeutet.

5. Verwendung einer pharmazeutischen Zusammensetzung nach Anspruch 1 oder 2, wobei Q eine ggf. substituierte Benzo[b]furan-2-ylgruppe bedeutet.

6. Verwendung einer pharmazeutischen Zusammensetzung nach Anspruch 5, wobei es sich bei den Substituenten um 1 bis 3 Halogenatome handelt.

7. Verwendung von Hydantoinderivaten der Formel (I) 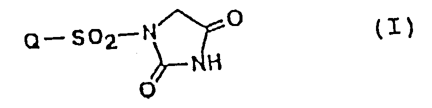 der nicht-toxischen Salze, Solvate oder Solvate von nicht-toxischen Salze davon, wobei Q die gleiche Bedeutung wie in Anspruch 1 hat, zur Herstellung eines Arzneipräparats oder Medikaments zur Therapie zur Verringerung von Hyperlipidspiegeln und/oder Hyperglucosespiegeln im Blut.

## Revendications

1. Utilisation d'une composition pharmaceutique comprenant au moins un des dérivés d'hydantoïne représentés par la formule (I) : 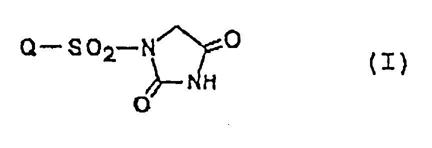 des sels non toxiques, des produits de solvatation ou des produits de solvatation des sels non toxiques de celui-ci; dans laquelle Q représente un groupe alkyle ayant 1 à 8 atomes de carbone, un groupe cycloalkyle ayant 3 à 6 atomes de carbone, un groupe biphénylyle, un groupe hétérocyclique monocyclique ou un groupe hétérocyclique condensé pouvant être substitué par un ou plusieurs substituants qui sont identiques ou différents et choisis dans le groupe constitué d'un atome d'halogène, d'un groupe alkyle ayant 1 à 4 atomes de carbone, d'un groupe nitro, d'un groupe cyano, d'un groupe carboxy protégé en option, d'un groupe carboxyméthyle protégé en option, d'un groupe haloalkyle ayant 1 à 4 atomes de carbone, d'un groupe alkylthio ayant 1 à 4 atomes de carbone, d'un groupe alkylcarbonyle dans lequel le groupe alkyle a 1 à 4 atomes de carbone, d'un groupe alcoxy ayant 1 à 4 atomes de carbone, d'un groupe alkylsulfinyle ayant 1 à 4 atomes de carbone, d'un groupe alkylsulfonyle ayant 1 à 4 atomes de carbone, d'un groupe hydroxy protégé en option, d'un groupe amino protégé en option, d'un groupe carbamoyle ou d'un groupe phényle,
ou un groupe : 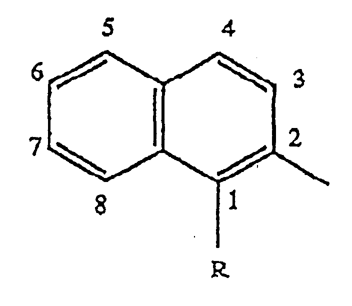 dans lequel R représente un atome de chlore ou de fluor, avec un support pharmaceutiquement acceptable, pour préparer un médicament pour le traitement et/ou la prévention du diabète sucré avec ou sans hyperlipidémie.

2. Utilisation d'une composition pharmaceutique comprenant au moins un des dérivés d'hydantoïne tels que définis en revendication 1, pour le traitement et/ou la prévention de l'hyperlipidémie.

3. Utilisation d'une composition pharmaceutique selon la revendication 1 ou 2, dans laquelle Q représente un groupe : 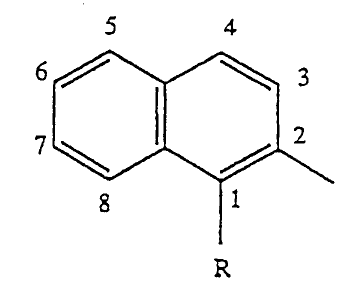 dans lequel R est tel que défini en revendication 1.

4. Utilisation d'une composition pharmaceutique selon la revendication 1 ou 2, dans laquelle Q représente un groupe benzo[b]thién-2-yle qui peut être substitué.

5. Utilisation d'une composition pharmaceutique selon la revendication 1 ou 2, dans laquelle Q représente un groupe benzo[b]furan-2-yle qui peut être substitué.

6. Utilisation d'une composition pharmaceutique selon la revendication 5, dans laquelle lesdits substituants sont 1 à 3 atomes d'halogène.

7. Utilisation des dérivés d'hydantoïne représentés par la formule (I) : 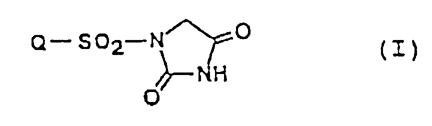 des sels non toxiques, des produits de solvatation ou des produits de solvatation des sels non toxiques de ceux-ci; dans laquelle Q a la même signification que celle définie en revendication 1 pour la fabrication d'une préparation médicale ou d'un médicament pour un traitement thérapeutique afin de réduire l'excès des taux de lipide et/ou de glucose dans le sang.
